# EUROPEAN PATENT SPECIFICATION

(11) **EP 0 505 397 B1**
(45) Date of publication and mention of the grant of the patent: **06.03.1996**
(21) Application number: 91900209.7
(22) Date of filing: 13.12.1990
(51) Int. Cl.: C12N 5/08

(54) **METHOD OF MAKING FACTOR-DEPENDENT HUMAN B CELL LINES**
VERFAHREN ZUR HERSTELLUNG VON FAKTORABHÄNGIGEN MENSCHLICHEN B-ZELLINIEN
PROCEDE DE FABRICATION DE LIGNEES DE LYMPHOCYTES B HUMAINS AVEC DEPENDANCE SELON LE FACTEUR

(30) Priority: 14.12.1989 EP 89403491
(43) Date of publication of application: 30.09.1992
(73) Proprietor: SCHERING-PLOUGH, F-92307 Levallois-Perret (FR)
(72) Inventor: BANCHEREAU, Jacques, F-69130 Ecully (FR); ROUSSET, Françoise, F-69005 Lyon (FR)
(74) Representative: Ritter, Stephen David
(86) International application number: EP9002195
(87) International publication number: WO9109115

(56) References cited:
- Biological Abstracts, vol. 88, 1989, A. Valle et al., see page AB-554, abstract 108680
- Biological Abstracts, vol. 36, 1989, A. Valle et al., see abstract 114250
- Biological Abstracts, vol. 87, no. 12, 1989, (Philadelphia, PA, US), J.L. Lasky et al., see pp. AB-646-647, abstract 127968
- Biological Abstracts, vol. 79, no. 11, 1985, (Philadelphia, PA, US), R. Frade et al., see p. AB-451, abstract 95695
- The Journal of Immunology, vol. 142, no. 5, 1 March 1989, The American Association of Immunologists, (US), J.B. Splawski et al., pages 1569-1575

## Description

### Field of the invention

The invention relates generally to the field of immunology, and more particularly, to methods of establishing longterm cultures of human lymphoid B cells capable of producing antibodies.

### BACKGROUND

Readily available hybridomas producing human monoclonal antibodies would give rise to valuable pharmaceutical and diagnostic compositions. Areas where human monoclonal antibodies may prove directly useful include passive immunization against viral and bacterial diseases, elimination of drugs and toxins, diagnostic imaging of neoplasms, targeting of drugs to tumors, and modulation of autoimmune disorders. Indirect utility of human monoclonal antibody-producing hybridomas lies with their use as a source of messenger RNA for making genetically engineered monoclonal antibodies in bacteria, or other non-human expression systems, e.g. Skerra et al, Science, Vol. 240, pgs. 1038-1041 (1988); and Moore et al, U.S. patent 4,642,334. Such methods have the great advantage of providing antibodies or binding compositions free of potentially dangerous human contaminants. Unfortunately, to date the use of human monoclonal antibodies in in vivo trials has been very limited, e.g. Burnett et al, in Strelkauskas, ed. Human Hybridomas: Diagnostic and Therapeutic Applications (Marcel Dekker, New York, 1987). A major stumbling block to progress in the field has been the inability to obtain long term and/or immortalized human B cell lines, e.g. James et al, J. Immunol. Meth., Vol. 100, pgs. 5-40 (1987); and Van Brunt, Biotechnology, Vol. 7, pgs. 561-563 (1989).

The availability of methods for routinely producing such lines, and methods of enriching and expanding antigen-specific subpopulations of B cells, would be a major breakthrough for the application of human monoclonal antibodies.

### SUMMARY OF THE INVENTION

The invention is directed to a method of establishing factor-dependent human B cell lines and antigen-specific subpopulations. The method includes the steps of isolating resting B cells or antigen-specific subpopulation carrying immunoglobulin of a desired specificity, and culturing the B cell or antigen-specific subpopulation in the presence of an agent capable of cross-linking its CD40 antigens. Preferably, the cross-linking agent is an immobilized monoclonal antibody specific for CD40. More preferably, the monoclonal antibody is immobilized on a solid phase or non-aqueous phase liquid substrate, such as microspheres, liposomes, or cellular membranes. Most preferably, the anti-CD40 monoclonal antibody is immobilized by culturing the B cell or antigen-specific subpopulation with non-replicating mammalian cells expressing the surface molecule, CDw32, also known as FcγRII, whenever the CD40-specific monoclonal antibody is of the IgG isotype. In this case, immobilization is achieved by the binding of the Fc portion of the antibody molecules with the Fc γ receptor. Longterm culturing of the B cell clone or subpopulation requires the continued presence of a cross-linking agent, and the culture growth rate is enhanced by the presence of the cytokines interleukin-2 (IL-2), interleukin-4 (IL-4), interleukin-6 (IL-6), and interferon- γ (INF-γ), either alone or in combination.

The invention also includes a method for producing Epstein-Barr virus (EBV)-transformed B cell lines wherein transformation takes place in the presence of a CD40 cross linking agent.

An important feature of the invention is the use of resting B cells as a starting material. These cells, as opposed to activated B cells, retain their surface-bound immunoglobulin, thereby rendering them amenable to antigen-specific selection.

### Brief description of the drawings

Figure 1A and 1B illustrate data on the growth of B cells on feeder layers of irradiated L cells expressing CDw32 with and without anti-CD40 monoclonal antibody and with and without IL-4.

Figure 2 illustrates the growth of resting B cells in response to anti-CD40 antibody presented in different ways.

### DETAILED DESCRIPTION OF THE INVENTION

Resting B cells for use in the invention can be obtained from a variety or sources by a variety of means, e.g. DiSabato et al. eds., Meth. in Enzymol., Vol. 108 (1984), and James et al (cited above). Preferably, B cells are obtained from peripheral blood, spleen, or tonsils. Most preferably, B cells are obtained from tonsils using the following technique: Tonsils are dissociated with wire mesh in phosphate buffered saline, pH 7.2, to obtain single cell suspensions. Mononuclear cells are separated by the standard Ficoll-Hypaque gradient method. To obtain purified B cell populations, T cells are removed from the mononuclear cells by twice rosetting with 2-aminoethylisothiouronium bromide treated sheep erythrocytes. Adherent cells (monocytes) are removed from the T cell-depleted mononuclear cells by incubating T cell-depleted mononuclear cells (in batches of approximately 2.5 x 10⁸ cells) in plastic flasks containing 25 ml RPMI 1640 with 10% fetal calf serum for 1 hour at 37°C. As determined by fluorescent activated cell sorting analysis the resulting preparation contains > 98% B cells, < 1% T cells, and < 1% monocytes. Resting B cells are obtained from this preparation by using a discontinuous gradient of Percoll (Pharmacia, Uppsala, Sweden) consisting of four solutions with densities of 1.075, 1.070, 1.060, and 1.055 g/ml. Resting B cells are recovered in the pellet, below the solution of Percoll of the highest density.

Further selection for antigen-specific subpopulation of resting B cells can be carried out by a variety of techniques including panning, rosetting, immunoadsorbent affinity chromatography, fluorescent-activated cell sorting (FACS), and the like. Casali et al, Science, Vol. 234, pgs. 476-479 (1986), describe the selection of antigen-specific B cells from peripheral blood by fluorescent-activated cell sorting. Briefly, the antigen of interest is biotinylated, incubated with the B cells, then with fluorescently labeled avidin. Cells having antibodies specific for the biotinylated antigen are sorted by the presence of the fluorescent label. Additional references describing FACS-based lymphocyte selection include Parks et al, Meth. Enzymol., Vol. 108, pgs. 197-241 (1984); and U.S. patent 4,325,706. Panning, immunoadsorbent affinity chromatography, and rosetting are described by Mage, Hubbard et al, and Haegert in Meth. Enzymol., Vol. 108, pgs. 118-124, 139-147, and 386-392, respectively (1984).

Monoclonal antibodies specific for CD40 are obtained by standard methods. Preferably, monoclonal antibodies G28-5 or Mab 89 are used as cross-linking agents. G28-5 is described by Ledbetter et al, J. Immunol., Vol. 138, pgs. 788-794 (1987) and in United Kingdom patent application N° 8713650, and the hybridoma cell line producing monoclonal antibodies G28-5 is deposited at the American Type Culture Collection (ATCC) (Rockville, MD) under accession number HB 9110. Mab 89 is described in Valle et al. Eur. J. Immunol., Vol. 19, pgs. 1463-1467 (1989), and the hybridoma cell line producing monoclonal antibodies Mab 89 has been deposited on September 14, 1989 with the European Collection of Animal Cell Cultures, PHLS Centre for Applied Microbiology and Research, Porton Down, Salisbury, Wilts. SP4 OJG, U.K. under accession number 89091401. Briefly, Mab 89 was obtained as follows. Eight week old BALB/c mice were injected i.p. four times at 3 week intervals with 5.0 x 10⁶ anti-IgM antibody activated tonsillar B cells. Three days after the last injection, spleen cells were collected and fused with NS1 myeloma cells (ratio 5:1) with the use of polyethylene glycol 1000 (Merck). After overnight incubation at 37°C in a 50 ml flask in complete RPMI 1640 medium containing 10% heat inactivated fetal bovine serum, 2mM glutamine, 100 IU/ml penicillin and 100 µg/ml streptomycin, the cell suspension was distributed in 24-well plates in medium supplemented with hypoxanthine and azaserine. Hybridoma supernatants were screened for their ability to bind to Jijoye cells, tonsil mononuclear cells and anti-IgM antibody activated B cells.

Several different substrates can be used to immobilize the anti-CD40 monoclonal antibodies, such as microspheres, erythrocytes, irradiated hybridomas expressing surface anti-CD40, and the like. Preferably, mammalian cell lines capable of stable expression of the FCγR are produced by co-transfecting a host mammalian cell with a vector carrying a selectable marker and a vector carrying a host-compatible promoter and a cDNA insert capable of encoding FcγRII. A cDNA clone carrying such an insert, pcD-hFcγR-16.2, is available from the ATCC under accession number 67565, and is described in Stuart et al, J.Exp. Med., Vol. 166, pgs. 1668-1684 (1987). The vector is similar to the pcD shuttle vector described by Okayama and Berg, Mol. Cell. Biol., Vol. 2, pgs. 161-170 (1982), and Vol. 3, pgs. 280-289 (1983), except that the SV40 promoter has been modified to improve expression by the downstream insertion of a portion of the long terminal repeat from a HTLV(I) retrovirus, as described by Takebe et al, Mol. Cell. Biol., Vol. 8, pgs. 466-472 (1988). The vector is conveniently propagated in E. coli K12 strain MC1061, described in J. Mol. Biol., Vol. 138, pg. 179 (1980).

For pcD-hFcγR-16.2, hosts include Chinese hamster ovary cells and mouse L cells, such as a thymidine kinase deficient mutant (tk⁻) L cell available from the American Type Culture Collection under accession number CCL 1.3. The selectable marker allows one to select host cells which have a high probability of containing the FcγR gene fully integrated into the host genome. Typically, the ratio of pcD-hfcγR-16.2 to the marker containing vector in the transfection solution is about 10:1, Thus, if the marker gene is integrated into the host genome, it is very likely that pcD-hFcγR-16.2 will also be integrated by virtue of its higher concentration. The selectable marker also provides a means of preventing the cultures of desired transformants from being overgrown by revertant cells. tk⁻ mouse L cells were cotransfected with pcD-hFcγR-16.2 and pSV2tk, a pSV2 plasmid carrying a thymidine kinase gene under control of the SV40 early promoter. The pSV2 is described in Mulligan et al., Science, Vol. 209, pgs. 1422-1427 (1980); Subramani et al. Mol. Cell. Biol., Vol. 1, pgs. 854-864 (1981); and is available from the American Type Culture Collection under accession number 37146. Both plasmids are amplified in E. coli, e.g. strain HB101 available from the ATCC under accession number 33694, and purified by cesium chloride equilibrium centrifugation. A suspension of about 1 x l0⁵ of tk⁻ L cells in 10 ml of Dulbecco's Modified Eagle medium (DME) with 10% fetal bovine serum is placed in a Falcon 3003 dish and cultured at 37°C for 20 hours in a 5% carbon dioxide gas incubator, after which the medium is replaced by 10 ml of fresh DME with 10% fetal bovine serum. The culture is incubated for an additional 4 hours. After incubation 0.5 ml of soluble A (50 mM Hepes, 280 mM NaCl, 1.5 mM sodium phosphate buffer, pH 7.22) and 0.5 ml of solution B (2M CaCl₂, 10 mg pcD-FcγR-16.2, 1 mg pSV2tk) are added to the culture medium, and the culture is incubated at 37°C for 24 hours in a 5% CO₂ atmosphere, after which the cells are placed in a selective medium with HAT (e.g. Sigma Chemical Co., St. Louis, MO). After two weeks the surviving colonies are subcloned by limiting dilution, and clones are assayed for expression of FcγR.

Preferably, IL-2, IL-4, IL-6, or INF-γ, either alone or in combination, is added to the B cell culture at a concentration of about 1 nanomolar. Alternatively, the concentration of IL-4 may be expressed in terms of units/ml, where units are defined as in Yokota et al, Proc. Natl. Acad. Sci., Vol. 83, pgs. 5894-5898 (1986). There a unit of IL-4 is defined as the amount of IL-4 required to cause half-maximal stimulation of tritiated thymidine uptake by 5 x 10³/200 µl T cells which were preactivated for 3 days with phytohemagglutinin and then extensively washed. Preferably, B cell cultures include about 100 U/ml of IL-4.

### EXAMPLES

### Example 1. Longterm Culture of Human B Cells Dependent on anti-CD40 Antibody and IL-4

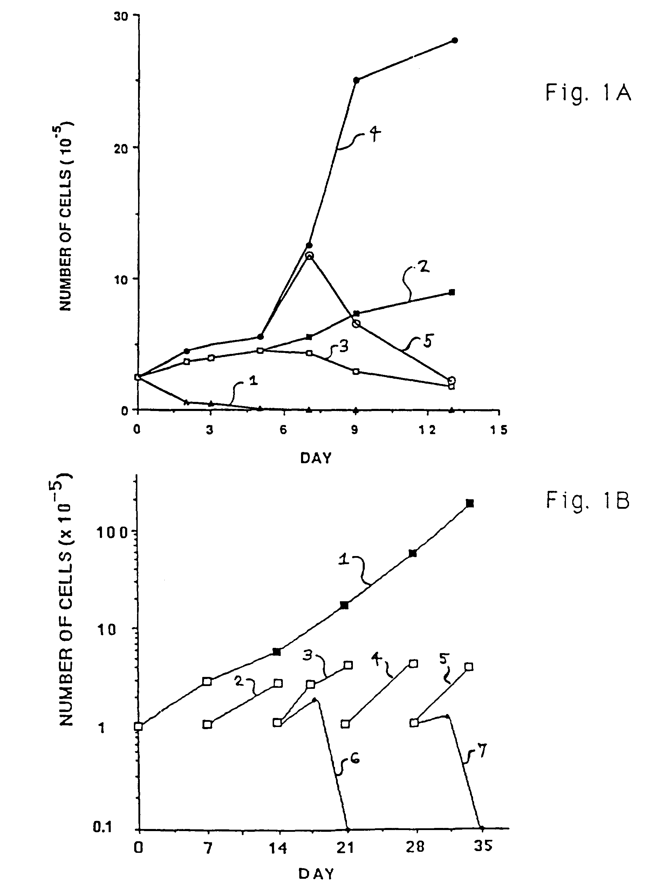

2x10⁵ purified spleen B cells in 500 µl complete culture medium were seeded in wells of 48-well microplates containing 2.5x10⁴ irradiated CDw32 L cells with 1 µg/ml anti-CD40 Mab 89 with or without 100 U/ml IL-4. Cultures incubated with either Mab 89 or Mab 89 plus IL-4 were divided at day 5 into two wells plated at 2.5x10⁴ irradiated CDw32 L cells with or without the original stimulant. Enumeration of viable B cells using Trypan Blue exclusion was carried out on a haemocytometer. Figure 1A shows the degree of B cell population growth for each culture condition. Cell numbers counted in one well at days 7, 9 and 13 have been doubled to take into account the splitting of the culture at day 5. The cell numbers have been evaluated in ten identical wells and the coefficient of variation was found to be less than 10%. Curve 1 of Fig. 1A illustrates the growth of B cells cultured on CDw32 L cells without Mab 89 and without IL-4. Curve 2 illustrates the growth of B cells cultured initially with 1 µg/ml Mab 89, then at day 5 the culture was split and half of the cells were transferred to another well with 2.5x10⁴ irradiated CDw32 L cells with 1 µg/ml Mab 89. Curve 3 illustrates the growth of B cells cultured initially with 1 µg/ml Mab 89, then at day 5 the culture was split and half of the cells were transferred to another well with 2.5x10⁴ irradiated CDw32 L cells without Mab 89. Curve 4 illustrates the growth of B cells cultured initially with 1 µg/ml Mab 89 and 100 U/ml IL-4, then at day 5 the culture was divided and half of the cells were transferred to another well with 2.5x10⁴ irradiated CDw32 L cells and 1 µg/ml Mab 89 and 100 U/ml IL-4. Curve 5 illustrates the growth of B cells cultured initially with 1 µg/ml Mab 89 and 100 U/ml IL-4, then at day 5 the culture was divided and half of the cells were transferred to another well with 2.5x10⁴ irradiated CDw32 L cells but without either Mab 89 or IL-4. The enhancing effect of CD40 cross linking in the presence of IL-4 is clearly seen.

In a separate experiment, purified tonsillar B cells were cultured at 10⁵ cells/ml on irradiated CDw32 L cells and 100 U/ml IL-4 in 500 µl of complete medium in wells of 48-well microplates. Cells were enumerated at the indicated times shown on Fig. 1B and cultures were re-initiated at the end of each week by seeding 10⁵B cells into new wells containing freshly irradiated CDw32 L cells, and fresh medium containing 1 µg/ml Mab 89 and 100 U/ml IL-4. Curve 1 illustrates the theoretical B cell population size if depleted culture media did not have to be replaced. Curves 2 through 5 illustrate the actual B cell populations in the initial culture and in the re-initiated cultures. Curves 6 and 7 show the decline in population size when a sample of B cell culture is re-initiated without Mab 89.

### Example 2 Further enhancement of B cell growth by IL-6 and IFN-γ

The growth enhancing effects of various cytokines were tested. 5x10³ purified tonsillar B cells were cultured on 5x10³ irradiated CDw32 L cells with 1 µg/ml Mab 89 in conical microwells. Tritiated thymidine uptake (after a 16 h pulse with 1 µCi) was assayed at the time points indicated in the table below. Each value in the table is a means of triplicate determinations. Cytokine concentrations were 25 U/ml IL-4; 2.5 IU/ml IL-1; 20 IU/ml IL-2; 50 U/ml IL-6; and 1000 IU/ml IFN-γ. A strong synergistic growth-inducing effect is seen between IL-4 and IFN-γ at day 8.

**Table I**

| Cytokine(s) Added | Tritiated Thymidine Uptake (cpm x 10⁻³) | | | |
|---|---|---|---|---|
| | Day 2 | Day 4 | Day 6 | Day 8 |
| -- | 1.5 | 5.3 | 7.0 | 5.1 |
| IL-4 | 3.1 | 7.9 | 13.3 | 21.8 |
| IL-2 | 2.3 | 4.4 | 12.7 | n.d. |
| IL-6 | 1.1 | 9.3 | 25.0 | 13.0 |
| IFN-γ | 4.6 | 6.7 | 16.6 | 14.0 |
| IL-4 + IL-2 | 1.8 | 13.2 | 18.6 | 18.4 |
| IL-4 + IL-6 | 1.9 | 17.8 | 22.8 | 32.0 |
| IL-4 + IFN-γ | 4.9 | 21.5 | 34.1 | 60.9 |

### Example 3. Antibody production by B cells stimulated with Mab 89 presented on CDw32-transfected L cells

2.5x10⁵ purified B cells were cultured with or without 2.5x10⁴ irradiated CDw32 L cells, with or without 0.5 µg/ml Mab 89, and with or without 100 U/ml IL-4. Supernatants were harvested at day 8 and immunoglobulin concentrations were determined by ELISA. The results for the various isotypes are shown in Table II.

**Table II**

| Culture Conditions | Concentration (ng/ml) | | | |
|---|---|---|---|---|
| | IgG | IgA | IgM | IgE |
| Control | 80 | 20 | 125 | <0.2 |
| Mab 89 | 225 | 70 | 380 | - - |
| IL-4 | 100 | <10 | 80 | <0.2 |
| Mab 89 + IL-4 | 105 | 30 | 170 | <0.2 |
| L cells | 210 | 10 | 290 | <0.2 |
| L cells + IL-4 | 650 | 200 | 490 | <0.2 |
| L cells + Mab 89 | 1800 | 40 | 320 | <0.2 |
| L cells+ Mab 89 + IL-4 | 5825 | 650 | 31200 | 458 |

### Example 4. Stimulation of B cell growth by irradiated hybridoma 89 cells

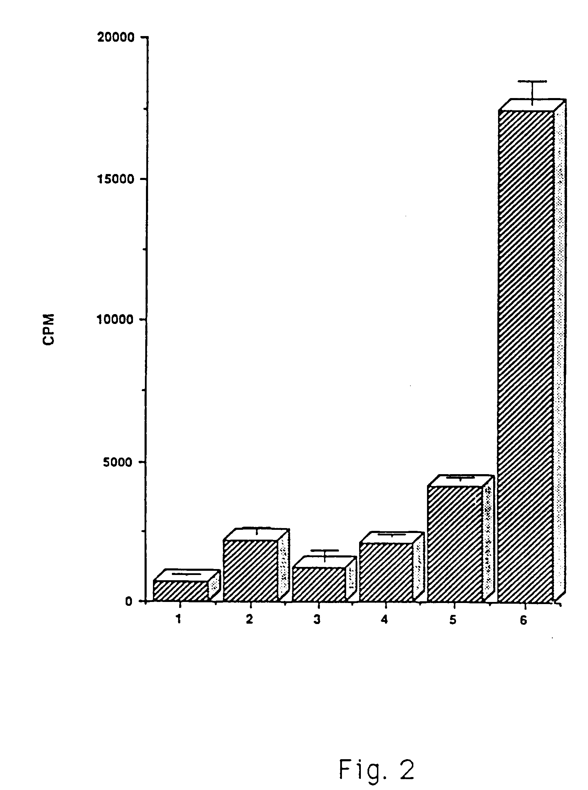

The growth-inducing effect of a hybridoma expressing surface anti-CD40 antibody was tested. Six experimental conditions were examined; and columns 1 to 6 of Figure 2 correspond to the conditions listed as follows: a control consisting of microtiter plate wells seeded with 10⁵ purified B cells (column 1); a control consisting of microtiter plate wells seeded with 10⁵ purified B cells and 1 µg/ml Mab 89 (column 2); a control consisting of microtiter plate wells seeded with 10⁴ irradiated hybridoma 89.1.4 cells (this is a derivative of hybridoma 89 which expresses antibody on its surface) (column 3); a control consisting of microtiter plate wells seeded with 10⁵ purified B cells and 10⁴ cells of an irradiated and unrelated hybridoma (column 4); a control consisting of microtiter plate wells seeded with 10⁵ purified B cells and 10⁴ irradiated hybridoma 89.1.4 cells (column 5); and a control consisting of microtiter plate wells seeded with 10⁵ purified B cells and 10⁴ irradiated CDw32 transfected L cells and 1 µg/ml of Mab 89 (column 6). Cell growth under the respective conditions was assayed by tritiated thymidine incorporation. The results are shown in Figure 2. The immobilized anti-CD40 on irradiated hybridoma 89.1.4 is nearly as effective as that on CDw32 transfected L cells in inducing growth in resting B cells.

### Example 5. Enhancement of EBV infection of B cells by Mab 89

An important way to establish antibody-producing human B cell lines is by infecting the B cells with Epstein Barr virus (EBV), e.g. James, Scand. J. Immunol., Vol. 29, pg. 257 (1989). However, the infection procedure is very inefficient, e.g. Stein et al. Cell. Immunol., Vol. 79, pg. 309 (1983). It was discovered that the presence of a CD40 cross-linking agent increases the efficiency of B cell infection. Infection efficiency was measured as a function of initial B cell numbers and the presence or absence of Mab 89, IL-4, or IL-6. Cultures were performed in 48-well (flat bottoms) plates for 105-cell cultures (each also containing 2.5x10⁴ irradiated CDw32 L cells) and in 96-well (round bottoms) plate for 100-cell and single cell colonies (each also containing 5.10³ irradiated CDw32 L cells). Factor concentrations were as follows: 1 µg/ml of Mab 89, 100 U/ml of IL-4, and 50 U/ml of IL-6. Table III lists the fraction of wells from which continuous EBV-transformed B cell lines were obtained for the various starting conditions and culture conditions.

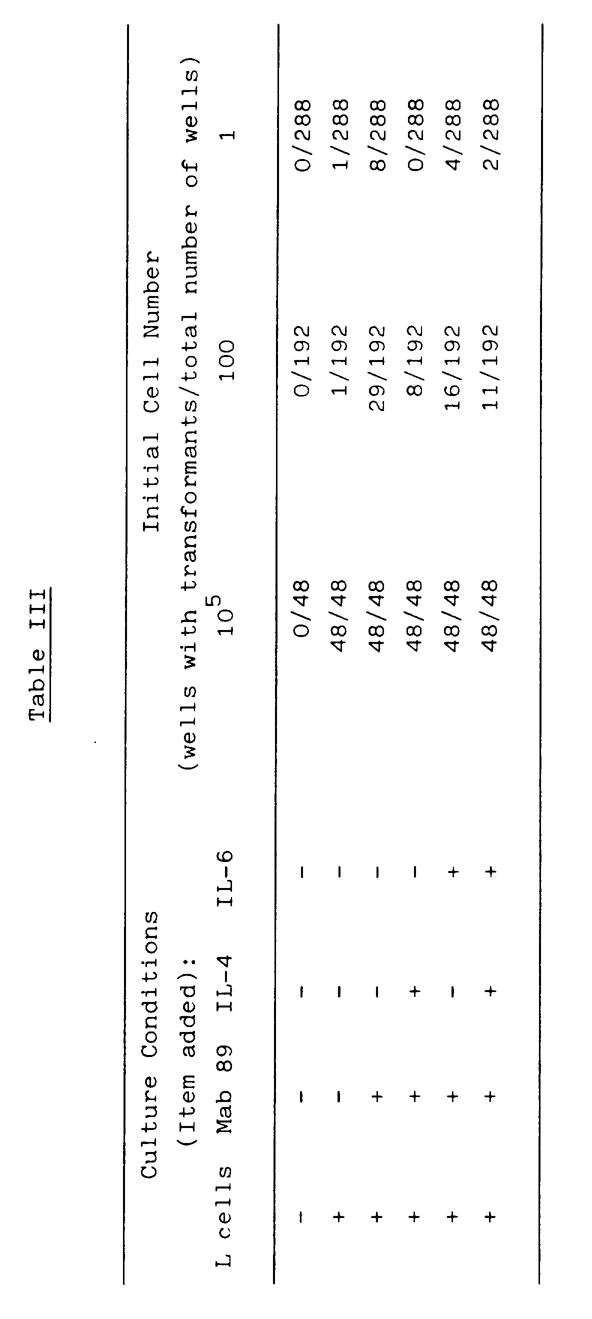

Besides enhanced EBV infection, the Mab 89/L cell system also affects antibody production by the EBV infected B cells. 10⁵ resting B cells were cultured on 2.5x10⁴ irradiated L cells expressing CDw32 and 1 µg/ml Mab 89 and EBV. Cells were enumerated at day 8 and immunoglobulin levels were measured by ELISA. Table IV lists the results and shows that the cells produce very high levels of IgE under these conditions.

**Table IV**

| Culture conditions | Cell Number x 10⁵ | Concentration (ng/ml) | | | |
|---|---|---|---|---|---|
| | | IgG | IgA | IgM | IgE |
| L cells + EBV | 0.45 | 667 | 105 | 959 | <0.2 |
| L cells + Mab 89 + EBV | 3.7 | 1245 | 639 | 1717 | <0.2 |
| L cells + Mab 89 + EBV +IL-4 | 6 | 683 | 87 | 572 | 478 |

### Example 6. Establishment of Human B Cell Clone Producing Rh Factor-Specific Monoclonal Antibodies

An important clinical use of anti-Rh antibodies is their injection into Rh-negative women shortly after the delivery of an Rh-positive infant to prevent the development of indigenous anti-Rh antibodies by the women which could be harmful to infants of subsequent pregnancies, e.g. Crookston, pgs. 601-608, in Rose et al, eds., Manual of Clinical Laboratory Immunology, 3rd Ed. (American Society for Microbiology, Washington, D.C., 1986). Besides the cost and difficulty of obtaining suitable donors for the anti-Rh immunoglobulin, a frequent danger associated with such injections is the transmission of blood-borne diseases such hepatitis, AIDS, and the like. In view of this problem, an in vitro source of anti-Rh antibodies would be highly desirable.

Resting B lymphocytes are isolated from an Rh-negative donor having serum containing anti-Rh antibody. A subpopulation of resting B cells carrying anti-Rh surface immunoglobulin may be isolated by repeated panning of the isolated B lymphocytes by the technique described by Mage (cited above), modified in the following manner: Instead of using a tissue culture dish coated with antibody specific for the anti-Rh antibody, the tissue culture dish is coated with anti-biotin antibody, and prior to addition to the tissue culture dish the isolated B lymphocytes are incubated with biotinylated Rh antigen. Cells binding the biotinylated Rh antigen are then isolated by panning as described by Mage (cited above). Alternatively, the resting B lymphocytes are isolated by fluorescent-activated cell sorting (FACS) using fluorescently labeled Rh antigen, or by rosetting following standard procedures, e.g. Elliott et al, Meth. Enzymol., Vol. 108, pgs 49-64 (1984).

The isolated anti-Rh resting B cells are then distributed among the wells (about 10⁵ B cells per well) of a microtiter plate, each well having previously been seeded with about 10⁴ CDw32-transfected, irradiated L cells, and each well ccntaining 0.4 ml of medium (RPMI 1640 with 10% fetal calf serum, and 2 mM glutamine) further containing 0.5 mg/ml of Mab 89 and 100 U/ml of IL-4. Cultures are expanded into larger containers and harvested for anti-Rh antibody after several weeks.

## Claims

1. A method for inducing human B cell proliferation, the method comprising: culturing over a period of at least 5 days human B cells in the presence of cross-linking cells having on their surface a monoclonal antibody that cross-links CD40 antigens, thereby resulting in a net increase in the number of B cells.

2. The method of Claim 1 wherein the number of said human B cells increases in culture over a period of at least 8 days.

3. The method of Claim 1 wherein the number of said human B cells increases in culture over a period of at least 13 days.

4. The method of Claim 1 wherein the number of said human B cells increases in culture over a period of at least 25 days.

5. The method of any preceding claim wherein said cross-linking cells carry an immobilized monoclonal antibody specific for said CD40 antigen.

6. The method of Claim 5 wherein said immobilized monoclonal antibody specifically binds to FcγRII receptors expressed by non-replicating mammalian cells.

7. The method of Claim 6 wherein said monoclonal antibody is a CD40-specific antibody having characteristics of monoclonal antibody 89 or G28-5 and wherein said non-replicating mammalian cells are mouse L cells expressing FcγRII receptors on their surface.

8. A method according to any preceding claim wherein the culturing of the B cells is carried out in the presence of interleukin-4.

9. A method according to any preceding claim wherein the culturing of the B cells is carried out in the presence of interferon-γ.

10. A method of producing Epstein-Barr virus-transformed human B cells, the method comprising the step of culturing human B cells in the presence of Epstein-Barr virus and an agent capable of cross-linking CD40 antigens.

11. The method of Claim 10 wherein said agent is an immobilized monoclonal antibody specific for said CD40 antigen.

12. The method of Claim 11 wherein said immobilized monoclonal antibody is attached to FcγRII receptors expressed by non-replicating mammalian cells.

13. The method of Claim 12 wherein said monoclonal antibody is selected from the group consisting of Mab 89 and G28-5 (ECACC 89091401 and ATCC HB9110) and wherein said non-replicating mammalian cells are mouse L cells stably transformed by pcD-hFcγR-16.2 (ATCC 67565).

14. Proliferating human B cells whose CD40 antigens are cross-linked by cross-linking cells having on their surface a monoclonal antibody that binds to said CD40 antigens.

15. The human B cells of Claim 14 wherein said cross-linking cells have on their surface an immobilized monoclonal antibody specific for said CD40 antigen.

16. The human B cells of Claim 15 wherein said immobilized monoclonal antibody specifically binds to FcγRII receptors expressed by non-replicating mammalian cells.

17. The human B cells of Claim 16 wherein said monoclonal antibody is a CD40-specific antibody having the characteristics of monoclonal antibody 89 or G28-5 and wherein said non-replicating mammalian cells are mouse L cells expressing FcγRII on their surface.

18. Epstein-Barr-virus-transformed human B cells whose CD40 antigens are cross-linked by an agent that binds to said CD40 antigens.

19. Epstein-Barr-virus-transformed human B cells of Claim 18 wherein said agent comprises immobilized monoclonal antibodies specific for said CD40 antigen.

20. Epstein-Barr-virus-transformed human B cells of Claim 8 wherein said agent comprises immobilized monoclonal antibodies bound to FcγRII receptors expressed by non-replicating mammalian cells.

21. Epstein-Barr-virus-transformed human B cells of Claim 20 wherein said monoclonal antibody is a CD40-specific antibody having the characteristics of monoclonal antibody 89 or G28-5 and wherein said non-replicating mammalian cells are mouse L cells expressing FcγRII receptors on their surface.

22. A method of producing human monoclonal antibodies which comprises the steps of cultivating cells as claimed in any one of Claims 14 to 21, and harvesting the monoclonal antibodies produced by said cells.

## Patentansprüche

1. Verfahren zum Induzieren der Proliferation von Human-B-Zellen, wobei das Verfahren umfaßt:
Kultivieren von Human-B-Zellen während wenigstens 5 Tagen in Gegenwart vernetzender Zellen, die auf ihrer Oberfläche einen monoklonalen Antikörper aufweisen, der CD40-Antigene vernetzt, was zu einer Nettozunahme der Zahl von B-Zellen führt.

2. Verfahren gemäß Anspruch 1, wobei die Zahl der Human-B-Zellen in Kultur im Verlaufe von wenigstens 8 Tagen zunimmt.

3. Verfahren gemäß Anspruch 1, wobei die Zahl der Human-B-Zellen in Kultur im Verlaufe von wenigstens 13 Tagen zunimmt.

4. Verfahren gemäß Anspruch 1, wobei die Zahl der Human-B-Zellen in Kultur im Verlaufe von wenigstens 25 Tagen zunimmt.

5. Verfahren gemäß einem der vorangehenden Ansprüche, wobei die vernetzenden Zellen einen immobilisierten monoklonalen Antikörper tragen, der für das CD40-Antigen spezifisch ist.

6. Verfahren gemäß Anspruch 5, wobei der immobilisierte monoklonale Antikörper spezifisch an FcγRII-Rezeptoren bindet, die von nichtreplizierenden Säugerzellen exprimiert werden.

7. Verfahren gemäß Anspruch 6, wobei der monoklonale Antikörper ein CD40-spezifischer Antikörper ist, der Merkmale des monoklonalen Antikörpers 89 oder G28-5 aufweist, und wobei die nichtreplizierenden Säugerzellen Maus-L-Zellen sind, die auf ihrer Oberfläche FcγRII-Rezeptoren exprimieren.

8. Verfahren gemäß einem der vorangehenden Ansprüche, wobei das Kultivieren der B-Zellen in Gegenwart von Interleukin-4 durchgeführt wird.

9. Verfahren gemäß einem der vorangehenden Ansprüche, wobei das Kultivieren der B-Zellen in Gegenwart von γ-Interferon durchgeführt wird.

10. Verfahren zur Herstellung von Human-B-Zellen, die durch Epstein-Barr-Virus transformiert sind, wobei das Verfahren den Schritt des Kultivierens von Human-B-Zellen in Gegenwart von Epstein-Barr-Virus und eines Mittels, das CD40-Antigene vernetzen kann, umfaßt.

11. Verfahren gemäß Anspruch 10, wobei das Mittel ein immobilisierter monoklonaler Antikörper ist, der für das CD40-Antigen spezifisch ist.

12. Verfahren gemäß Anspruch 11, wobei der immobilisierte monoklonale Antikörper an FcγRII-Rezeptoren gebunden ist, die von nichtreplizierenden Säugerzellen exprimiert werden.

13. Verfahren gemäß Anspruch 12, wobei der monoklonale Antikörper aus der Gruppe ausgewählt ist, die aus Mab 89 und G28-5 (ECACC 89091401 und ATCC HB9110) besteht, und wobei die nichtreplizierenden Säugerzellen Maus-L-Zellen sind, die durch pcD-hFcγR-16.2 (ATCC 67565) stabil transformiert sind.

14. Proliferierende Human-B-Zellen, deren CD40-Antigene durch vernetzende Zellen vernetzt sind, die auf ihrer Oberfläche einen monoklonalen Antikörper aufweisen, der an die CD40-Antigene bindet.

15. Human-B-Zellen gemäß Anspruch 14, wobei die vernetzenden Zellen auf ihrer Oberfläche einen immobilisierten monoklonalen Antikörper aufweisen, der für das CD40-Antigen spezifisch ist.

16. Human-B-Zellen gemäß Anspruch 15, wobei der immobilisierte monoklonale Antikörper spezifisch an FcγRII-Rezeptoren bindet, die von nichtreplizierenden Säugerzellen exprimiert werden.

17. Human-B-Zellen gemäß Anspruch 16, wobei der monoklonale Antikörper ein CD40-spezifischer Antikörper ist, der Merkmale des monoklonalen Antikörpers 89 oder G28-5 aufweist, und wobei die nichtreplizierenden Säugerzellen Maus-L-Zellen sind, die auf ihrer Oberfläche FcγRII exprimieren.

18. Human-B-Zellen, die durch Epstein-Barr-Virus transformiert sind und deren CD40-Antigene durch ein Mittel vernetzt sind, das an die CD40-Antigene bindet.

19. Human-B-Zellen, die durch Epstein-Barr-Virus transformiert sind, gemäß Anspruch 18, wobei das Mittel immobilisierte monoklonale Antikörper umfaßt, die für das CD40-Antigen spezifisch sind.

20. Human-B-Zellen, die durch Epstein-Barr-Virus transformiert sind, gemäß Anspruch 18, wobei das Mittel immobilisierte monoklonale Antikörper umfaßt, die an FcγRII-Rezeptoren gebunden sind, die von nichtreplizierenden Säugerzellen exprimiert werden.

21. Human-B-Zellen, die durch Epstein-Barr-Virus transformiert sind, gemäß Anspruch 20, wobei der monoklonale Antikörper ein CD40-spezifischer Antikörper ist, der Merkmale des monoklonalen Antikörpers 89 oder G28-5 aufweist, und wobei die nichtreplizierenden Säugerzellen Maus-L-Zellen sind, die auf ihrer Oberfläche FcγRII-Rezeptoren exprimieren.

22. Verfahren zur Herstellung menschlicher monoklonaler Antikörper, das die Schritte des Kultivierens von Zellen gemäß einem der Ansprüche 14 bis 21 und des Gewinnens der von den Zellen erzeugten monoklonalen Antikörper umfaßt.

## Revendications

1. Méthode pour induire la prolifération de cellules B humaines, la méthode comprenant :
la mise en culture sur une période d'au moins 5 jours de cellules B humaines en présence de cellules réticulantes ayant à leur surface un anticorps monoclonal qui réticule les antigènes de CD40, avec ainsi pour résultat une augmentation nette du nombre des cellules B.

2. Méthode de la revendication 1 où le nombre desdites cellules β humaines augmente en culture sur une période d'au moins 8 jours.

3. Méthode selon la revendication 1 où le nombre desdites cellules β humaines augmente en culture sur une période d'au moins 13 jours.

4. Méthode selon la revendication 1 où le nombre desdites cellules B humaines augmente en culture selon une période d'au moins 25 jours.

5. Méthode selon toute revendication précédente où lesdites cellules réticulantes portent un anticorps monoclonal immobilisé spécifique dudit antigène de CD40.

6. Méthode de la revendication 5 où ledit anticorps monoclonal immobilisé se lie aux récepteurs FcγRII exprimés par des cellules mammaliennes ne se répliquant pas.

7. Méthode de la revendication 6 où ledit anticorps monoclonal est un anticorps spécifique de CD40 ayant les caractéristiques de l'anticorps monoclonal 89 ou G28-5 et où lesdites cellules mammaliennes ne se répliquant pas sont des cellules L de souris exprimant les récepteurs FcγRII à leur surface.

8. Méthode selon toute revendication précédente où la mise en culture des cellules B est effectuée en présence d'interleukine-4.

9. Méthode selon toute revendication précédente où la mise en culture des cellules B est effectuée en présence d'interféron-γ.

10. Méthode de production de cellules B humaines transformées par le virus d'Epstein-Barr, la méthode comprenant l'étape de cultiver des cellules B humaines en présence du virus d'Epstein-Barr et d'un agent capable de réticuler les antigènes de CD40.

11. Méthode de la revendication 10 où ledit agent est un anticorps monoclonal immobilisé spécifique dudit antigène de CD40.

12. Méthode de la revendication 11 où ledit anticorps monoclonal immobilisé est attaché aux récepteurs FcγRII exprimés par les cellules mammaliennes ne se répliquant pas.

13. Méthode de la revendication 12 où ledit anticorps monoclonal est sélectionné dans le groupe consistant en Mab 89 et G28-5 (ECACC 89091401 et ATCC HB9110) et où lesdites cellules mammaliennes ne se répliquant pas sont des cellules L de souris transformées de manière stable par pcD-hFcγR-16.2 (ATCC 67565).

14. Cellules B humaines en prolifération dont les antigènes de CD40 sont réticulés par des cellules réticulantes ayant à leur surface un anticorps monoclonal qui se lie auxdits antigènes de CD40.

15. Cellules B humaines de la revendication 14 où lesdites cellules réticulantes ont, sur leur surface, un anticorps monoclonal immobilisé spécifique dudit antigène de CD40.

16. Cellules B humaines de la revendication 15 où ledit anticorps monoclonal immobilisé se lie spécifiquement aux récepteurs FcγRII exprimés par des cellules mammaliennes ne se répliquant pas.

17. Cellules B humaines de la revendication 16 où ledit anticorps monoclonal est un anticorps spécifique de CD40 ayant les caractéristiques de l'anticorps monoclonal 89 ou G28-5 et où lesdites cellules mammaliennes ne se répliquant pas sont des cellules L de souris exprimant à leur surface Fc γRII.

18. Cellules B humaines transformées par le virus d'Epstein-Barr dont les antigènes de CD40 sont réticulés par un agent qui se lie auxdits antigènes de CD40.

19. Cellules B humaines transformées par le virus d'Epstein-Barr de la revendication 18 où ledit agent comprend des anticorps monoclonaux immobilisés spécifiques dudit antigène de CD40.

20. Cellules B humaines transformées par le virus d'Epstein-Barr de la revendication 18 où ledit agent comprend des anticorps monoclonaux immobilisés liés aux récepteurs Fcγ RII exprimés par les cellules mammaliennes ne se répliquant pas.

21. Cellules B humaines transformées par le virus d'Epstein-Barr de la revendication 20 où ledit anticorps monoclonal est un anticorps spécifique de CD40 ayant les caractéristiques de l'anticorps monoclonal 89 ou G28-5 et où lesdites cellules mammaliennes ne se répliquant pas sont des cellules L de souris exprimant les récepteurs FcγRII à leur surface.

22. Méthode de production d'anticorps monoclonaux qui comprend les étapes de cultiver des cellules selon l'une quelconque des revendications 14 à 21 et de récolter les anticorps monoclonaux produits par lesdites cellules.
